(19) Europäisches Patentamt
European Patent Office
Office européen des brevets

(11) Publication number: **0 057 998**
**B1**

(12) # EUROPEAN PATENT SPECIFICATION

(45) Date of publication of patent specification: **08.08.84**

(51) Int. Cl.³: **C 07 C 103/52, A 61 K 37/02**

(21) Application number: **82300300.9**

(22) Date of filing: **21.01.82**

(54) **Alicyclic compounds, their production and use.**

(30) Priority: **23.01.81 JP 9470/81**
**09.06.81 JP 88539/81**
**22.12.81 JP 208817/81**

(43) Date of publication of application:
**18.08.82 Bulletin 82/33**

(45) Publication of the grant of the patent:
**08.08.84 Bulletin 84/32**

(84) Designated Contracting States:
**BE CH DE FR GB IT LI NL SE**

(56) References cited:
EP - A - 0 003 056
EP - A - 0 012 401

(73) Proprietor: **Takeda Chemical Industries, Ltd.**
**27, Doshomachi 2-chome Higashi-ku**
**Osaka-shi Osaka, 541 (JP)**

(72) Inventor: **Oka, Yoshikazu**
**69, Hagiharadaihigashi 1-chome**
**Kawanishi Hyogo 666 (JP)**
Inventor: **Nishikawa, Kohei**
**5-19, Oharano-kamisatotorimicho**
**Nishikyo-ku Kyoto 610-11 (JP)**
Inventor: **Miyake, Akio**
**30-22, Ominemotomachi 2-chome**
**Hirakata Osaka 573 (JP)**

(74) Representative: **Lewin, John Harvey et al,**
**Elkington and Fife High Holborn House 52/54**
**High Holborn**
**London WC1V 6SH (GB)**

**EP 0 057 998 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel alicyclic compounds, which are useful as pharmaceuticals.

As compounds having hypotensive effect due to inhibitory activity to angiotensin converting enzyme, there are known various amino acid derivatives [e.g. those disclosed in Japanese Patent Unexamined Publication (kokai) Nos. 77—116457, 77—136117, 79—12372, 80—38382, 80—81845 and 81—18953, which correspond to USP—4105776, USP—4053651, British Unexamined Pub. No. 2000508, European Unexamined Pub. Nos. 9183 and 12401, and USP—4,256,761, respectively.

For example, EP—A1—0012401 discloses compounds of the formula:

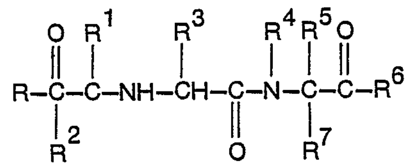

in which R and $R^6$ are inter alia hydroxy or alkoxy, $R^1$ is inter alia hydrogen, alkyl or aralkyl, $R^2$ and $R^7$ are hydrogen or alkyl, $R^3$ is inter alia hydrogen, alkyl or phenylalkyl, $R^4$ is hydrogen or lower alkyl, and $R^5$ is inter alia hydrogen.

The novel compounds of the present invention are not only different structurally from any of those known compounds but also have desirable angiotensin converting enzyme inhibitory activity and hypotensive actions.

This invention is thus concerned with alicyclic compounds of the formula:

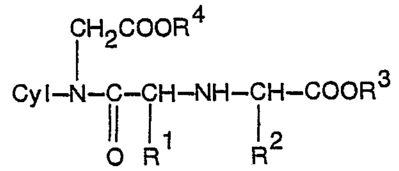

(I)

wherein Cyl is an alicyclic group containing up to 10 carbon atoms; $R^1$ is hydrogen, lower alkyl or aralkyl; $R^2$ is hydrogen, lower alkyl, or aralkyl which may optionally be substituted; and $R^3$ and $R^4$ are the same or different and each is hydrogen or lower alkyl, and salts thereof.

Referring to the above formula (I), the alicyclic group Cyl includes monocyclic and polycyclic alicyclic groups of 3 to 10 carbon atoms, and each alicyclic group may contain one or two double bonds. The monocyclic alicyclic groups may for example be $C_{3-10}$ cycloalkyl (e.g. cyclopropyl, cyclobutyl, cyclopentyl, cyclohexyl, cycloheptyl, cyclooctyl, cyclodecyl, $C_{3-10}$ cycloalkenyl (e.g. 2-cyclopropenyl, 3-cyclopentenyl, 1-cyclohexenyl, 1,4-cyclohexadienyl, 4-cycloheptenyl), etc. and said polycyclic alicyclic groups may for example be such bicyclic groups as norbornyl, bicyclo[2.2.2]octyl, pinanyl, 5-norbornen-2-yl, bicyclo[3.3.1]non-9-yl, bicyclo[4.3.0]non-8-yl, bicyclo[3.3.0]oct-7-yl, etc. and such tricyclic groups as adamantyl.

The lower alkyl group $R^1$ includes $C_{1-4}$ alkyl (e.g. methyl, ethyl, propyl, isopropyl, butyl, isobutyl, sec-butyl, tert-butyl). The aralkyl group $R^1$ includes phenyl-lower($C_{1-2}$)alkyl containing 7 to 8 carbon atoms, such as benzyl, phenethyl, etc.

The lower alkyl group $R^2$ includes $C_{1-4}$ alkyl similar to $R^1$ and the aralkyl group $R^2$ includes phenyl-lower($C_{1-4}$)alkyl of 7 to 10 carbon atoms, such as benzyl, phenethyl, 3-phenylpropyl, $\alpha$-methylbenzyl, $\alpha$-ethylbenzyl, $\alpha$-methylphenethyl, $\beta$-methylphenethyl, $\beta$-ethylphenethyl, etc. and the phenyl moiety of such phenyl-lower alkyl group may have 1 to 3 substituent groups, examples of which include halogens (fluorine, chlorine, bromine, iodine), $C_{1-4}$ alkyls (e.g. methyl, ethyl, propyl butyl), $C_{1-4}$ alkoxys (e.g. methoxy, ethoxy, propoxy, butoxy, methylenedioxy, isopropoxy), hydroxyl and so on.

The lower alkyl groups $R^3$ and $R^4$ may be similarly exemplified to those of $R^1$.

In the above-mentioned compounds, preferred embodiments are those of the formula (I) wherein Cyl represents $C_{3-10}$ alicyclic group; $R^1$ is hydrogen, $C_{1-4}$ alkyl or phenyl-$C_{1-2}$ alkyl; $R^2$ is hydrogen, $C_{1-4}$ alkyl or phenyl-$C_{1-4}$ alkyl which is unsubstituted or substituted by one to three of halogen, $C_{1-4}$ alkyl, $C_{1-4}$ alkoxy and hydroxyl; and $R^3$ and $R^4$ independently represent hydrogen or $C_{1-4}$ alkyl, and pharmaceutically acceptable salts thereof.

Among the compounds (I), further preferred are compounds in which Cyl is a $C_{4-7}$ saturated monocyclic alicyclic group or a polycyclic alicyclic group selected from norbornyl, bicyclo[4.3.0]non-8-yl, and adamantyl; $R^1$ is methyl; $R^2$ is phenethyl; $R^3$ is $C_{1-4}$ alkyl; and $R^4$ is hydrogen.

As said salts of compound (I), there may be mentioned pharmaceutically acceptable salts such as inorganic acid salts (e.g. hydrochloride, hydrobromide, sulfate, nitrate, phosphate) and organic acid

salts (e.g. acetate, tartrate, citrate, fumarate, maleate, toluenesulfonate, methanesulfonate), etc. When either one of $R^3$ and $R^4$ is hydrogen, there may further be mentioned metal salts, such as salts with sodium, potassium, calcium, aluminum, etc. and salts with bases such as triethylamine, guanidine, ammonium, hydrazine, guinine, cinchonine, etc.

The compound (I) of this invention can be produced, for example, by subjecting a compound of the formula:

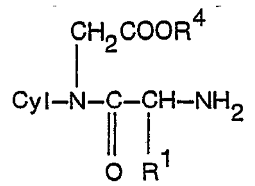

(II)

wherein Cyl, $R^1$ and $R^4$ are as defined hereinbefore, and a compound of the formula:

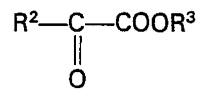

(III)

wherein $R^2$ and $R^3$ are as defined hereinbefore, to condensation reaction under reductive conditions.

The reductive conditions mentioned above may for example be the conditions for catalytic reduction in the presence of such a metal catalyst as platinum, palladium, Raney nickel, rhodium or the like as either supported on a suitable supporting medium or unsupported; conditions for reduction with a metal hydride such as lithium aluminum hydride, lithium borohydride, lithium cyanoborohydride, sodium borohydride, sodium cyanoborohydride or the like; conditions for reduction with a metal such as sodium metal or magnesium metal and an alcohol; conditions for reduction with a metal such as iron or zinc and an acid such as hydrochloric acid or acetic acid; conditions for electrolytic reduction; or conditions for reduction with a reductase. The above reaction is generally conducted in the presence of water or an organic solvent (e.g. methanol, ethanol, ethyl ether, dioxane, methylene chloride, chloroform, benzene, toluene, dimethylformamide, dimethylacetamide, etc.). The reaction temperature is generally within the range of about −20°C to +100°C, although it depends on the reductive conditions employed. While this reaction can be conducted satisfactorily at atmospheric pressure, it may also be conducted at superatmospheric or subatmospheric pressure. In order that the dehydrative condensation of (II) and (III), which is likely to be a first phase of the present reaction, may be carried through successfully, a dehydrating agent such as anhydrous magnesium sulfate, anhydrous sodium sulfate or a molecular sieve may be added to the reaction system.

The compound (I) in which $R^3$ and/or $R^4$ is hydrogen, i.e. a free dicarboxylic or monocarboxylic acid, can also be produced for example by hydrolysis of a corresponding ester compound. Catalytic reduction may be an alternative procedure when the ester is the benzyl or diphenylmethyl ester, for instance. The hydrolysis of such an ester compound may be carried out under the conventional hydrolytic conditions, for example, permitting an acid (e.g. hydrochloric acid, sulfuric acid, hydrobromic acid, p-toluenesulfonic acid, trifluoroacetic acid, etc.) or a base (e.g. sodium hydroxide, potassium hydroxide, sodium carbonate, potassium carbonate, triethylamine, etc.) to act on the ester in water or an organic solvent such as an alcohol (e.g. methanol, ethanol, etc.) or an ether (e.g. tetrahydrofuran, dioxane, etc.) or a mixture thereof at a temperature within the range of about −10° to +150°C. On the other hand, the catalytic reduction can be carried out with the aid of a catalyst such as platinum, palladium-on-carbon, Raney nickel or the like in the presence of water or an organic solvent or a mixture thereof. Although this reaction proceeds satisfactorily at atmospheric temperature and pressure, it may also be conducted at elevated pressure or/and elevated temperature conditions, if necessary.

The compound (I) can also be produced by subjecting a compound of the formula:

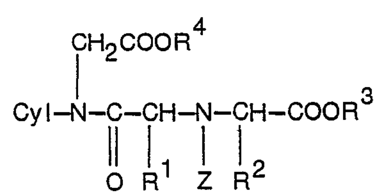

(IV)

wherein Cyl, $R^1$, $R^2$, $R^3$ and $R^4$ are as defined hereinbefore; and Z is a protective group which can be eliminated by hydrolysis or catalytic reduction, to hydrolysis or catalytic reduction. As the protective group that can be eliminated by hydrolysis, as designated by Z in (IV), there may be employed substantially all kinds of acyl groups as well as trityl, although benzyloxycarbonyl, tert-butoxycarbonyl,

trifluoroacetyl, trityl, etc., in particular, are suitable for ensuring comparatively mild reaction conditions. The protective group that can be eliminated by catalytic reduction, as also designated by Z, may for example be benzyl, diphenylmethyl or benzyloxycarbonyl. The hydrolysis reaction in this process is conducted in water or an organic solvent such as methanol, ethanol, dioxane, pyridine, acetic acid, acetone or methylene chloride, or a mixture thereof, with or without addition of an accelerator agent such as an acid (e.g. hydrochloric acid, hydrobromic acid, hydroiodic acid, hydrofluoric acid, sulfuric acid, methanesulfonic acid, p-toluenesulfonic acid, trifluoroacetic acid, etc.) or a base (e.g. sodium hydroxide, potassium hydroxide, potassium carbonate, sodium hydrogen carbonate, sodium acetate, triethylamine, etc.). The reaction temperature may generally be about −10 to +150°C. The catalytic reduction in this process is conducted in water or an organic solvent (e.g. methanol, ethanol, dioxane, tetrahydrofuran, etc.), or a mixture thereof and in the presence of a suitable catalyst such as platinum, palladium-on-carbon, etc. This reaction is conducted at atmospheric pressure or an elevated pressure of about 150 kg/cm² at the maximum and at a temperature that may range from room temperature to about 150°C, although the reaction generally proceeds satisfactorily even at atmospheric temperature and pressure.

The compound (I) can also be produced by reacting a compound (II) with a compound of the formula:

$$X-\overset{R^2}{\overset{|}{C}}H-COOR^3 \qquad (V)$$

wherein $R^2$ and $R^3$ are as defined hereinbefore; and X is halogen or a group of the formula $R^5SO_2-O-$ in which $R^5$ is lower $(C_{1-4})$alkyl, phenyl or p-tolyl. This reaction proceeds as the two reactant compounds are maintained in a suitable solvent at a temperature between −10° and +150°C. For the purpose of accelerating the reaction, a base such as potassium carbonate, sodium hydroxide, sodium hydrogen carbonate, pyridine, triethylamine or the like may be added to the reaction system.

The product compound (I) of this invention can be isolated from the reaction mixture by per se conventional separation and purification procedures such as extraction, concentration, filtration, recrystallization, column chromatography and thin-layer chromatography.

The compound (I) may be occuring in two or more stereosiomeric forms depending on the species of alicyclic group Cyl and also on the presence or absence of substituents $R^1$ and $R^2$. Such isomers, severally or as a mixture, also fall within the scope of this invention and, if desired, each of the isomers may be produced independently. For example, by conducting the above-mentioned reactions using one of the isomers of starting compounds (II) and (IV), there can be produced one of the isomers of compound (I). Or when the product compound is a mixture of two or more isomers, it can be resolved or otherwise separated into the individual isomers by per se conventional procedures, for example through the formation of salts with optically active acids (e.g. camphorsulfonic acid, tartaric acid, dibenzoyltartaric acid, etc.) or optically active bases (e.g. cinchonine, cinchonidine, quinine, quinidine, α-methylbenzylamine, dehydroabietylamine, etc.), chromatography, fractional recrystallization, etc. When such isomers are asymmetrical with respect to carbon atoms carrying substituents $R^1$ and $R^2$, the isomer having an S-configuration tends to be more satisfactory as far as the following pharmacological properties are concerned.

The above-described alicyclic compounds (I) and salts according to this invention show such biological activities as inhibitory activities to angiotensin converting enzyme, bradykinin decomposing enzyme (Kininase) and enkephalinase in animals, especially in mammals (e.g. man, dog, cat, rabbit, guinea pig, rat, etc.), and are of value as diagnostic prophylactic and therapeutic drugs for hypertension or as analgesics or analgesic synergists. These compounds are low-toxic, absorbed well on oral administration and stable enough and, therefore, for such clinical applications as mentioned above, can be safely administered orally or parenterally, either as they are or as formulated with suitable pharmaceutically acceptable carrier, excipients or diluents, in such dosage forms or as such pharmaceutical compositions as powders, granules, tablets, capsules, injections, etc. The dosage depends on the patient's condition and the route of administration to be used. When the compound of this invention or said composition thereof is administered to an adult patient for the treatment of hypertension, the proper dosage is generally about 0.02 to 20 mg/kg per dose or preferably about 0.2 to 2 mg/kg per dose for oral administration and about 0.002 to 0.2 mg/kg or preferably about 0.02 to 0.2 mg/kg for intravenous injection. About 2 to 5 doses are preferably given daily according to the patient's condition.

The following reference, working and experimental examples are given to illustrate this invention in further detail but should by no means be construed as limiting the scope of the invention.

Reference Example 1

In 200 ml of methanol were dissolved 10 g of cyclopentanone and 21.6 g of glycine ethyl ester hydrochloride, and under stirring at room temperature, 7.48 g of sodium cyanoborohydride was added portionwise. The mixture was stirred at room temperature for 3 hours and concentrated under reduced

pressure. To the residue was added 500 ml of water, and the mixture was adjusted to pH 10 with dilute aqueous sodium hydroxide and extracted with 300 ml of ethyl acetate. The extract was washed with saturated aqueous sodium chloride and dried over sodium sulfate. The solvent was then distilled off under reduced pressure. The residual oil was dissolved in 200 ml of ether, and to the solution was added 10 ml of 10% alcoholic HCl, whereupon crystals separated out. The crystals were collected by filtration to give 12.7 g of N-cyclopentylglycine ethyl ester hydrochloride as colorless needles melting at 174—175°C.

Reference Example 2

To a solution of 11.4 g of N-carbobenzoxy-L-alanine and 6.7 ml of triethylamine in 100 ml of tetrahydrofuran was added 6.5 ml of isobutyl chlorocarbonate portionwise under stirring at −10°C. To this solution, at −10 to −5°C, was added a solution of 10 g of N-cyclopentylglycine ethyl ester hydrochloride and 6.7 ml of triethylamine in 100 ml of chloroform. The mixture was allowed to stand at room temperature overnight and filtered. The filtrate was concentrated and, following addition of 300 ml of water to the residue, the mixture was extracted with 200 ml of ethyl acetate. The ethyl acetate layer was washed with 5% hydrochloric acid, aqueous sodium hydrogen carbonate and water in that order and dried over sodium sulfate. The solvent was then distilled off and the residual oil was separated and purified by column chromatography on 150 g of silica gel, using toluene-ethyl acetate (5:1) as the eluent to afford 11 g of N-carbobenzoxy-L-alanyl-N-cyclopentylglycine ethyl ester as an oil. To a solution of 10 g of this oil in 100 ml of ethanol was added 16 ml of 2N sodium hydroxide and the mixture was stirred at room temperature for 5 hours. The reaction mixture was concentrated, followed by addition of 100 ml of water. The mixture was acidified with 10% hydrochloric acid and the oil that was liberated thereby was extracted with 300 ml of ethyl acetate. The extract was washed with water, dried and concentrated to give 8 g of N-carbobenzoxy-L-alanyl-N-cyclopentylglycine. This oil (8 g) was dissolved in 100 ml of ethanol and then in 20 ml of water and catalytic reduction was carried out in the presence of 4 g of 5% palladium-on-carbon. After completion of the absorption of hydrogen, the catalyst was filtered off and the filtrate was concentrated under reduced pressure. To the residue was added 10 ml of ethanol and the resultant crystals were collected by filtration to give 2.9 g of L-alanyl-N-cyclopentylglycine melting at 171—172°C.

Elemental analysis:

Calcd. for $C_{10}H_{18}N_2O_3$ : C, 56.05; H, 8.47; N, 13.08
Found : C, 55.96; H, 8.45; N, 12.98

Reference Example 3

To a solution of 39.2 g of cyclohexanone and 14 g of glycine ethyl ester hydrochloride in 300 ml of methanol was added 10 g of sodium cyanoborohydride at room temperature over an hour, and then the mixture was stirred at that temperature for 3 hours. The insolubles were filtered off with the aid of Celite and the filtrate was concentrated. After addition of 200 ml of saturated aqueous sodium hydrogen carbonate, the concentrate was extracted with 300 ml of ethyl acetate, dried over sodium sulfate and concentrated. The residual oil was dissolved in ether and 10% alcoholic HCl was added, whereupon crystals separated out. The crystals were dissolved in 200 ml of dichloromethane and filtered once. The filtrate was concentrated and ether was added to the residue to give 17 g of N-cyclohexylglycine ethyl ester hydrochloride as crystals melting at 157—160°C.

NMR spectrum ($CDCl_3$): 2.6—1.0 (13H, m, cyclohexane ring protons and $OCH_2CH_3$), 3.0—3.7 (1H, m, $\overset{|}{-CH}-\overset{|}{N}-$), 3.90 (2H, s, $NHCH_2CO-$), 4.35 (2H, q, $OCH_2CH_3$).

(*s* denotes a singlet, *d* a doublet, *t* a triplet, *q* a quartet and *m* multiplet. The same applies in the following reference examples and examples.)

Reference Example 4

To a solution of 10.1 g of N-carbobenzoxy-L-alanine and 4.6 g of triethylamine in 100 ml of tetrahydrofuran was added 6.2 g of isobutyl chlorocarbonate portionwise under stirring at −5 to −15°C. The mixture was stirred for an hour and, following addition of a solution of 10 g of N-cyclohexylglycine ethyl ester hydrochloride and 4.6 g of triethylamine in 100 ml of chloroform at −10°C, the mixture was stirred at room temperature overnight. The reaction mixture was filtered and the filtrate was concentrated. To the residue was added water and the mixture was extracted with ethyl acetate. The extract was washed with dilute hydrochloric acid, aqueous sodium hydrogen carbonate and water in that order and dried over sodium sulfate. The solvent was then distilled off and the residual oil was separated and purified by silica gel column chromatography, using toluene-ethyl acetate (5:1) as the

eluent to afford 13.1 g of N-carbobenzoxy-L-alanyl-N-cyclohexlglycine ethyl ester as an oil. In 100 ml of ethanol was dissolved 11.4 g of this oil and, following addition of 16 ml of 2N sodium hydroxide, the mixture was stirred at room temperature for 4 hours. It was concentrated and, after addition of water, the insolubles were removed by extraction with ethyl acetate. The aqueous layer was acidifed with dilute hydrochloric acid and the substance that was liberated thereby was extracted with ethyl acetate. The extract was washed with water, dried and concentrated to give 8.8 g of N-carbobenzoxy-L-alanyl-N-cyclohexylglycine as an oil. This oil was dissolved in 80 ml of ethanol and 40 ml of water and, following addition of 4 g of 5% palladium-on-carbon, the solution was stirred in a hydrogen gas stream at room temperature. The catalyst was then filtered off and the filtrate was concentrated to give 4.3 g of L-alanyl-N-cyclohexylglycine as crystals melting at 164—165°C.

Reference Example 5

30 Grams of cycloheptanone, 48.5 g of glycine ethyl ester and 16.7 g of sodium cyanoborohydride were reacted and worked up in the same manner as Reference Example 1. By the procedure there was obtained 44 g of N-cycloheptylglycine ethyl ester hydrochloride as colorless needles melting at 179—180°C.

Elemental analysis:

| | |
|---|---|
| Calcd. for $C_{11}H_{21}NO_2{\cdot}HCl$ | : C, 56.03; H, 9.41; N, 5.94; Cl, 15.04 |
| Found | : C, 55.79; H, 9.53; N, 5.86; Cl, 15.00 |

Reference Example 6

10 Grams of cyclobutanone, 16 g of glycine ethyl ester and 6 g of sodium cyanoborohydride were reacted and worked up in the same manner as Reference Example 1. By the procedure there was obtained 11 g of N-cyclobutylglycine ethyl ester hydrochloride as colorless needles melting at 156—159°C.

Reference Example 7

40 Grams of N-carbobenzoxy-L-alanine and 40 g of N-cycloheptylglycine ethyl ester hydrochloride were reacted and worked up in the same manner as Reference Example 2. By the procedure there was obtained 8.5 g of L-alanyl-N-cycloheptylglycine as colorless crystals melting at 147—148°C.

Elemental analysis:

| | |
|---|---|
| Calcd. for $C_{12}H_{22}N_2O_3{\cdot}1/4H_2O$ | : C, 58.39; H, 9.19; N, 11.35 |
| Found | : C, 58.37; H, 9.23; N, 11.13 |

Reference Example 8

4 Grams of N-carbobenzoxy-L-alanine and 4 g of N-cyclobutylglycine ethyl ester hydrochloride were reacted and worked up in the same manner as Reference Example 2. By the procedure was obtained 1 g of L-alanyl-N-cyclobutylglycine as a colorless amorphous powdery product.

Elemental analysis:

| | |
|---|---|
| Calcd. for $C_9H_{16}N_2O_3$ | : C, 53.98; H, 8.06; N, 13.99 |
| Found | : C, 53.69; H, 8.12; N, 14.08 |

Reference Example 9

The procedure of Reference Example 2 was repeated except that 49 g of N-carbobenzoxy-L-phenylalanine and 30 g of N-cyclopentylglycine ethyl ester hydrochloride were used. The procedure gave 14.7 g of L-phenylalanyl-N-cyclopentylglycine as a colorless amorphous powdery product.

IR spectrum $\nu_{max}^{KBr}cm^{-1}$: 3230, 1640 (broad), 1442, 1320, 1310, 1180.

Reference Example 10

In 200 ml of ethanol were dissolved 25 g of 2-exonorbornylamine and 25 g of benzaldehyde and catalytic reduction was carried out at atmospheric pressure and temperature using 5% palladium-on-carbon as the catalyst. When the absorption of hydrogen ceased, the catalyst was filtered off and the filtrate was distilled off under reduced pressure. To the residue were added 100 ml of 20% ethanolic hydrochloric acid and 200 ml of ethyl acetate to give 30 g of 2-exobenzylaminonorborane hydrochloride as colorless flakes melting at 220—225°C.

Reference Example 11

To 500 ml of methyl ethyl ketone were added 30 g of 2-exobenzylaminonorborane hydrochloride and 19.1 g of tert-butyl chloroacetate and, following addition of 33 g of potassium iodide and 50 g of potassium carbonate, the mixture was refluxed under stirring for 2 hours. After cooling, 500 ml of water

was added and the reaction mixture was extracted with 800 ml of ethyl acetate. The organic layer was washed with 200 ml of 1% aqueous phosphoric acid and water in that order and dried over anhydrous magnesium sulfate. The solvent was then distilled off under reduced pressure to give 35 g of N-benzyl-N-(2-exonorbornyl)glycine tert-butyl ester as an oil. This product was dissolved in 200 ml of methanol and catalytic reduction was carried out at room temperature, using 10% palladium-on-carbon as catalyst. When hydrogen was no longer absorbed, the catalyst was filtered off and the methanol was distilled off under reduced pressure. To the residue was added 300 ml of ether, followed by dropwise addition of 300 ml of 0.35 N HCl in ether. By the above procedure was obtained 22.5 g of N-(2-exonorbornyl)glycine tert-butyl ester hydrochloride as colorless flakes melting at 180—183°C (decomp.).

Reference Example 12

13 Grams of N-(2-exonorbornyl)glycine tert-butyl ester hydrochloride and 16.7 g of N-carbobenzoxy-L-alanine were reacted and worked up in the same manner as Reference Example 4 to give 21 g of N-carbobenzoxy-L-alanyl-N-(2-exonorbornyl)glycine tert-butyl ester as an oil. This product was dissolved in 200 ml of methanol and the solution was stirred with 4.7 g of oxalic acid and 4 g of 10% palladium-on-carbon in a hydrogen gas stream at room temperature. The catalyst was then filtered off and the filtrate was concentrated under reduced pressure. To the residue was added 300 ml of ether to give 14 g of L-alanyl-N-(2-exonorbornyl)glycine tert-butyl ester oxalate as colorless crystals melting at 115—120°C.

$[\alpha]_D^{25}$ −7.1° (c = 1 in methanol).

Reference Example 13

In the same manner as Reference Example 1, 4.2 g of 2-adamantanone and 7.2 g of glycine tert-butyl ester phosphite were reacted in the presence of sodium cyanoborohydride to give 6.4 g of N-(2-adamantylglycine tert-butyl ester as an oil.

IR spectrum $\nu_{max}^{neat}$ cm$^{-1}$: 1730 (C=O)
NMR spectrum ($CDCl_3$) $\delta$: 1.5 (s, 9H), 1.5—2.2 (m, 14H), 2.7 (m, 1H), 3.3 (s, 2H).

Reference Example 14

In the same manner as Reference Example 4, 6.4 g of N-(2-adamantyl)glycine tert-butyl ester was reacted with 8.1 g of N-carbobenzoxy-L-alanine to give 8 g of N-carbobenzoxy-L-alanyl-N-(2-adamantyl)glycine tert-butyl ester as an oil.

IR spectrum $\nu_{max}^{neat}$ cm$^{-1}$: 3300 (NH), 1700 (C=O), 1640 (C=O).
NMR spectrum ($CDCl_3$) $\delta$: 1.1—1.5 (m, 12H), 1.5—2.3 (m, 14H), 3.9—4.4 (m, 4H), 5.1 (s, 2H), 7.3 (s, 5H).

Reference Example 15

Using 8 g of N-carbobenzoxy-L-alanyl-N-(2-adamantyl)glycine tert-butyl ester, catalytic reduction was carried out in the same manner as Reference Example 12 in the presence of oxalic acid and palladium-on-carbon as catalyst. The above procedure gave 1.2 g of L-alanyl-N-(2-adamantyl)glycine tert-butyl ester oxalate as a colorless amorphous powdery product.

IR spectrum $\nu_{max}^{Nujol}$ cm$^{-1}$: 1710, 1700, 1640, 1600 (C=O).

Reference Example 16

A mixture of 143 g of ethyl 3-phenylpropionate, 234 g of ethyl oxalate and 154 ml of 28% ethanolic sodium ethoxide solution was heated on a water bath at 60—70°C for 1.5 hours, with the ethanol being distilled off under reduced pressure. To the resulting red syrup was added 1.8 l of 15 V/V% sulfuric acid and the mixture was refluxed under stirring for 15 hours. The resulting oil was separated, neutralized with 10% sodium hydroxide and extracted with ethyl acetate. The aqueous layer was acidified with dilute sulfuric acid. The resulting oil was extracted with ethyl acetate, washed with water and dried. The solvent was then distilled off under reduced pressure to give 130 g of 2-oxo-4-phenylbutyric acid as an oil.

Reference Example 17

To a mixture of 650 ml of ethanol and 13 ml of conc. sulfuric acid was added 130 g of 2-oxo-4-phenylbutyric acid and the mixture was refluxed for 5 hours. The reaction mixture was concentrated to about half its original volume and diluted with 500 ml of water. The resulting oil was separated and the aqueous layer was extracted with ethyl acetate. The extract and the oil were combined and dried. The solvent was then distilled off under reduced pressure. Distillation of the residue under reduced pressure gave 113 g of ethyl 2-oxo-4-phenylbutyrate as a colorless oil boiling at 135—141°C/3 mm Hg.

Reference Example 18

In the same manner as Reference Example 17, 2-oxo-4-phenylbutyric acid was reacted with n-propanol to give a light-yellow oil of propyl 2-oxo-4-phenylbutyrate boiling at 105—118°C/1 mmHg.

Reference Example 19

In the same manner as Reference Example 17, 2-oxo-4-phenylbutyric acid was reacted with n-butanol to give a light-yellow oil of butyl 2-oxo-4-phenylbutyrate boiling at 145—150°C/4 mmHg.

Reference Example 20

To a solution of 99.6 g of veratraldehyde and 124.8 g of malonic acid in 240 ml of pyridine was added 7.5 ml of piperidine and the mixture was warmed at 80—85°C for an hour and then at 110—115°C for 3 hours. After cooling, the mixture was poured into an excess of water and the crystals that separated out were collected by filtration. The crystals were dissolved in dilute aqueous sodium hydroxide and then acidified with HCl to give 70 g of 3,4-dimethoxycinnamic acid as needles melting at 182—183°C. In 500 ml of ethanol were dissolved 35 g of the above needles and the solution was saturated with hydrogen chloride gas, after which it was allowed to stand at room temperature overnight. The ethanol was then distilled off and the residual crystals were dissolved in ethyl acetate and washed with dilute aqueous sodium hydrogen carbonate and water in that order and dried. The ethyl acetate was distilled off and the residual crystals were recrystallized from ethanol to give 35 g of ethyl 3,4-dimethoxycinnamate as flakes melting at 53—55°C. In 300 ml of ethanol was dissolved 34 g of this product and following addition of 10 g of 5% palladium-on-carbon the mixture was shaken in a hydrogen gas stream at room temperature for 3 hours. The catalyst was then filtered off and the filtrate was concentrated to give 34 g of ethyl 3,4-dimethoxyphenylpropionate as an oil. Under stirring at 60°C, 34.1 g of this oil and 43 g of diethyl oxalate were added to a sodium ethoxide solution prepared from 3.8 g of sodium metal and 150 ml of ethanol. After completion of addition, the mixture was further stirred at 70—75°C for 3 hours. The ethanol was distilled off under reduced pressure and following addition of 200 ml of water the ethyl acetate layer was removed. The aqueous layer was acidified with HCl and extracted with ethyl acetate. The ethyl acetate layer was washed with water, dried and concentrated to give 27 g of ethyl 2-oxo-3-ethoxycarbonyl-4-(3,4-dimethoxyphenyl)-butyrate as an oil. In a mixture of 80 ml dimethyl sulfoxide and 8 ml water was dissolved 26 g of this oil and, following addition of 6.3 g of sodium chloride, the mixture was stirred at 120°C for 2 hours. After cooling, an excess of water was added and the mixture was extracted with ethyl acetate. The extract was washed with water, dried and concentrated and the residual crystals were recrystallized from ether to give 15 g of ethyl 2-oxo-4-(3,4-dimethoxyphenyl)butyrate as prisms melting at 85—87°C.

Example 1

To a solution of 1 g of L-alanyl-N-cyclopentylglycine and 4.8 g of ethyl 2-oxo-4-phenylbutyrate in 40 ml of 50% ethanol was added portionwise a solution of 0.58 g of sodium cyanoborohydride in 10 ml of ethanol under stirring at room temperature. The mixture was allowed to stand overnight and then concentrated. After addition of 20 ml of water to the residue, the solution was adjuted to pH 9 with dilute aqueous sodium hydroxide and the insolubles were removed by extraction with ether. The aqueous layer was adjusted to pH 4 with 10% hydrochloric acid and extracted twice with 50 ml portions of ethyl acetate. The extract was washed with water, dried and concentrated. The residue was dissolved in 50 ml of ether, and 1 ml of 10% ethanolic hydrochloric acid was added. The oil separating out was washed with ether to give 0.7 g of N-(1-ethoxycarbonyl-3-phenylpropyl)-L-alanyl-N-cyclopentylglycine hydrochloride as a colorless powdery product.

NMR spectrum ($CDCl_3$) $\delta$: 1.20 (3H, t, $OCH_2CH_3$), 1.24 (3H, d, $—COCH(CH_3)NH—$), 1.65 (8H, m, cyclopentane ring protons), 4.20 (4H, m, $OCH_2CH_3$ and

$$\overset{|}{—N}CH_2COOH),$$ 7.30 (5H, s, aromatic ring protons).

IR spectrum $\nu_{max}^{Neat}$ $cm^{-1}$: 3650—2300 (COOH), 1740 (ester), 1660 (amide).

Example 2

To a solution of 5.5 g of L-alanyl-N-cyclohexylglycine and 20.6 g of ethyl 2-oxo-4-phenylbutyrate in 100 ml of 50% ethanol was added a solution of 2.5 g of sodium cyanoborohydride in 20 ml of ethanol dropwise under stirring at room temperature. After the mixture was stirred for 4 hours, the solvent was distilled off and to the residue was added 50 ml of water. The solution was adjusted to pH 10 with dilute aqueous sodium hydroxide and the substance that was liberated thereby was removed by extraction with ethyl acetate. The aqueous layer was adjusted to pH 4 with 1N HCl and extracted with 200 ml of ethyl acetate. The extract was washed with saturated aqueous sodium chloride and dried over sodium sulfate. The ethyl acetate was then distilled off and the residue was dissolved in 3 ml of 10% ethanolic hydrochloric acid and concentrated to dryness. The residue was washed three times

with ether to give 1.2 g of N-(1-ethoxycarbonyl-3-phenylpropyl)-L-alanyl-N-cyclohexylglycine hydrochloride as a colorless powdery product.

Elemental analysis:

| | |
|---|---|
| Calcd. for $C_{23}H_{34}N_2O_5 \cdot HCl$ | : C, 60.71; H, 7.75; N, 6.15 |
| Found | : C, 59.99; H, 7.71; N, 6.45 |

IR spectrum $\nu_{max}^{KBr}$ $cm^{-1}$: 3650—2300 (CO*OH*), 1738 (ester), 1645, 1655 (amide).

Example 3

The same reaction as Example 1 was carried out using 1.5 g of L-alanyl-N-cyclopentylglycine, 8 g of ethyl 2-oxo-4-(3,4-dimethoxyphenyl)butyrate and 0.88 g of sodium cyanoborohydride. The procedure gave 1.3 g of N-[1-ethoxycarbonyl-3-(3,4-dimethoxyphenyl)propyl]-L-alanyl-N-cyclopentylglycine hydrochloride as a colorless amorphous powdery product.

Elemental analysis:

| | |
|---|---|
| Calcd. for $C_{24}H_{36}N_2O_7 \cdot HCl$ | : C, 57.53; H, 7.44; N, 5.59 |
| Found | : C, 57.23; H, 7.24; N, 5.27 |

IR spectrum $\nu_{max}^{KBr}$ $cm^{-1}$: 3500—2300, 1732, 1640, 1506, 1440, 1258, 1010.

Example 4

The same reaction as Example 1 was carried out using 1.5 g of L-alanyl-N-cycloheptylglycine, 5.1 g of ethyl 2-oxo-4-phenylbutyrate and 0.77 g of sodium cyanoborohydride. The procedure gave 0.48 g of N-(1-ethoxycarbonyl-3-phenylpropyl)-L-alanyl-N-cycloheptylglycine hydrochloride as a colorless amorphous powdery product.

Elemental analysis:

| | |
|---|---|
| Calcd. for $C_{24}H_{36}N_2O_5 \cdot HCl$ | : C, 61.45; H, 7.95; N, 5.97 |
| Found | : C, 61.61; H, 8.13; N, 5.82 |

IR spectrum $\nu_{max}^{KBr}$ $cm^{-1}$: 3500—2300, 1740, 1640, 1450, 1200.

Example 5

The same reaction as Example 1 was carried out using 1.5 g of L-alanyl-N-cyclobutylglycine, 6 g of ethyl 2-oxo-4-phenylbutyrate and 0.8 g of sodium cyanoborohydride. The procedure gave 0.3 g of N-(1-ethoxycarbonyl-3-phenylpropyl)-L-alanyl-N-cyclobutylglycine hydrochloride as a colorless amorphous powdery product.

Elemental analysis:

| | |
|---|---|
| Calcd. for $C_{21}H_{30}N_2O_5 \cdot HCl$ | : C, 59.08; H, 7.32; N, 6.56 |
| Found | : C, 58.88; H, 7.14; N, 6.65 |

Example 6

The same reaction as Example 1 was carried out using 1.5 g of L-alanyl-N-cycloheptylglycine, 5.5 g of propyl 2-oxo-4-phenylbutyrate and 0.8 g of sodium cyanoborohydride. The procedure gave 0.55 g of N-(1-propoxycarbonyl-3-phenylpropyl)-L-alanyl-N-cycloheptylglycine hydrochloride as a colorless amorphous powdery product.

Elemental analysis:

| | |
|---|---|
| Calcd. for $C_{25}H_{38}N_2O_5 \cdot HCl$ | : C, 62.15; H, 8.14; N, 5.80 |
| Found | : C, 61.81; H, 8.29; N, 6.07 |

IR spectrum $\nu_{max}^{KBr}$ $cm^{-1}$: 3500—2300, 1740, 1640, 1450, 1200.

Example 7

The same reaction as in Example 1 was carried out using 1 g of L-alanyl-N-cycloheptylglycine and 4 g of butyl 2-oxo-4-phenylbutyrate. The procedure gave 0.6 g of N-(1-butoxycarbonyl-3-phenylpropyl)-L-alanyl-N-cycloheptylglycine hydrochloride as a colorless amorphous powdery product.

Elemental analysis:

| | |
|---|---|
| Calcd. for $C_{26}H_{40}N_2O_5 \cdot HCl$ | : C, 62.82; H, 8.31; N, 5.64 |
| Found | : C, 63.10; H, 8.52; N, 5.93 |

IR spectrum $\nu_{max}^{KBr}$ $cm^{-1}$: 3500—2300, 1730, 1640, 1442, 1200.

Example 8

To a suspension of 2 g of L-phenylalanyl-N-cyclopentylglycine and 7.1 g of ethyl 2-oxo-4-phenylbutyrate in 50 ml of ethanol was added portionwise a solution of 1.2 g of sodium cyanoborohydride in 30 ml of ethanol. After completion of addition, the mixture was stirred for an hour and concentrated. To the residue was added 150 ml of water and the insolubles were removed by extraction with ethyl acetate. The aqueous layer was adjusted to pH 4.0 with 1N hydrochloric acid and the substance that was liberated thereby was extracted with ethyl acetate. The extract was washed with saturated aqueous sodium chloride, dried and concentrated. The residue was dissolved in 50 ml of ether and, following addition of 1 ml of 10% ethanolic hydrochloric acid, the oil that was liberated thereby was triturated with ether to give 0.37 g of N-(1-ethoxycarbonyl-3-phenylpropyl)-L-phenylalanyl-N-cyclopentylglycine hydrochloride as a colorless amorphous powdery product.

IR spectrum $\nu_{max}^{KBr}$ $cm^{-1}$: 3500—2300, 1740, 1642, 1452, 1220.

Example 9

The same reaction as in Example 8 was carried out using 2 g of L-phenylalanyl-N-cyclopentylglycine, 4 g of ethyl pyruvate and 1.2 g of sodium cyanoborohydride. The procedure gave 0.35 g of N-(1-ethoxycarbonylethyl)-L-phenylalanyl-N-cyclopentylglycine hydrochloride as a colorless amorphous powdery product.

IR spectrum $\nu_{max}^{KBr}$ $cm^{-1}$: 3500—2300, 1740, 1640, 1450, 1210, 1190.

Example 10

In 80 ml of ethanol was suspended 2 g of L-alanyl-N-cyclopentylglycine and dissolved by addition of 0.5 g of sodium methoxide. The solution was adjusted to pH 5 with acetic acid and stirred with 5.8 g of ethyl oxo-4-phenylbutyrate, 4 g of Raney nickel and 10 g of Molecular Sieve 3A in a hydrogen gas stream at room temperature for 8 hours. The reaction mixture was filtered and the filtrate was concentrated under reduced pressure. After addition of 100 ml of water to the residue, the solution was adjusted to pH 8 with dilute sodium hydroxide and the insolubles were extracted with 50 ml of ether. The aqueous layer was adjusted to pH 3 with dilute hydrochloric acid and extracted with 100 ml of ethyl acetate. The extract was washed with water and dried over sodium sulfate. The solvent was then distilled off, and 2 ml of 10% ethanolic hydrochloric acid and 50 ml of ether were added to the residue, whereupon an oil separated out. This oil was washed three times with ether to give 0.9 g of powdery substance, which was crystallized from acetone to yield 0.32 g of N-[1-(S)-ethoxycarbonyl-3-phenylpropyl]-L-alanyl-N-cyclopentylglycine hydrochloride melting at 149—152°C, $[\alpha]_D^{22}$ −8° (c=0.5 in ethanol).

Elemental analysis:

| | |
|---|---|
| Calcd. for $C_{22}H_{32}N_2O_5 \cdot HCl$ | : C, 59.92; H, 7.54; N, 6.35 |
| Found | : C, 59.77; H, 7.68; N, 6.22 |

Example 11

The same reaction as in Example 10 was carried out using 2 g of L-alanyl-N-cyclopentylglycine and 5.2 g of propyl 2-oxo-4-phenylbutyrate. The procedure gave 0.6 g of N-[1-(S)-propoxycarbonyl-3-phenylpropyl]-L-alanyl-N-cyclopentylglycine hydrochloride as colorless crystals melting at 145—148°C, $[\alpha]_D^{22}$ −7° (c=0.5 in ethanol).

Elemental analysis:

| | |
|---|---|
| Calcd. for $C_{23}H_{34}N_2O_5 \cdot HCl$ | : C, 60.71; H, 7.75; N, 6.16 |
| Found | : C, 60.76; H, 7.73; N, 6.10 |

Example 12

Using 4 g of L-alanyl-N-cyclopentylglycine and 11 g of butyl 2-oxo-4-phenylbutyrate, there was synthesized 0.95 g of N-[1-(S)-butoxycarbonyl-3-phenylpropyl]-L-alanyl-N-cyclopentylglycine hydrochloride as colorless crystals melting at 138—140°C, $[\alpha]_D^{22}$ −4.6° (c=1 in ethanol).

Elemental analysis:

| | |
|---|---|
| Calcd. for $C_{24}H_{36}O_5N_2 \cdot HCl \cdot 1/4H_2O$ | : C, 60.87; H, 7.98; N, 5.91; Cl, 7.49 |
| Found | : C, 60.87; H, 7.94; N, 6.25; Cl, 7.68 |

Example 13

To a solution of 0.7 g of N-(1-ethoxycarbonyl-3-phenylpropyl)-L-alanyl-N-cyclopentylglycine hydrochloride in 15 ml of methanol was added 4 ml of 1N aqueous sodium hydroxide, and the mixture was stirred at room temperature for 4 hours. The reaction mixture was concentrated and, after addition of 5 ml of water, it was adjusted to pH 5 with 10% hydrochloric acid and left standing. The resulting crystals were filtered to give 0.28 g of N-(1-carboxy-3-phenylpropyl)-L-alanyl-N-cyclopentylglycine as prisms melting at 136—138°C.

Elemental analysis:
Calcd. for $C_{20}H_{28}N_2O_5 \cdot 1.5H_2O$ : C, 59.53; H, 7.74; N, 6.94
Found : C, 59.61; H, 7.60; N, 6.75

The mother liquor was adsorbed on 50 ml of an ion exchange resin (IR—120, $H^+$-form) eluted with 2% pyridine and concentrated to give an additional crop (0.34 g) of the above-mentioned compound.

Example 14

In the same manner as Example 13, 0.8 g of N-[1-ethoxycarbonyl-3-(3,4-dimethoxy)phenylpropyl]-L-alanyl-N-cyclopentylglycine hydrochloride as obtained in Example 3 was reacted to give 0.3 g of N-[1-(S)-carboxy-3-(3,4-dimethoxyphenyl)propyl]-L-alanyl-N-cyclopentylglycine as colorless crystals melting at 140—143°C, $[\alpha]_D^{22}$ +16° (c=0.5 in ethanol).

Elemental analysis:
Calcd. for $C_{22}H_{32}N_2O_7$ : C, 58.13; H, 7.54; N, 6.16
Found : C, 58.38; H, 7.24; N, 6.05

Example 15

To a solution of 5 g of L-alanyl-N-(2-exonorbornyl)glycine tert-butyl ester oxalate, in 100 ml of ethanol were added 1.1 g of sodium acetate, 2.5 g of acetic acid, 5 g of ethyl 4-phenyl-2-oxobutyrate and 10 g of Molecular Sieve 3A. This mixture was stirred with 5 g of Raney nickel (wet) in a hydrogen gas stream at room temperature for 8 hours. The insolubles were separated and the ethanol was distilled off. To the residue was added 100 ml of ethyl acetate, and the mixture was washed with saturated aqueous sodium hydrogen carbonate, dried over anhydrous magnesium sulfate and the solvent was distilled off. The residual oil was purified by silica gel chromatography [eluant: acetone-benzene (1:20)]. As the first eluate, 1 g of N-[1-(R)-ethoxycarbonyl-3-phenylpropyl]-L-alanyl-N-(2-exonorbornyl)glycine tert-butyl ester was obtained as an oil.

IR spectrum $\nu_{Neat}$ $cm^{-1}$: 1730, 1640 (C=O).

As the second eluate, there was obtained 2 g of N-[1-(S)-ethoxycarbonyl-3-phenylpropyl]-L-alanyl-N-(2-exonorbornyl)glycine tert-butyl ester as an oil.

IR spectrum $\nu_{Neat}$ $cm^{-1}$: 1730, 1640 (C=O).

Elemental analysis:
Calcd. for $C_{28}H_{42}N_2O_5$ : C, 69.10; H, 8.70; N, 5.76
Found : C, 68.87; H, 8.80; N, 6.10

$[\alpha]_D^{24}$ −22.6° (c=0.6 in methanol)
Mass spectrum m/e: 486 ($M^+$)

Example 16

In 2 ml of acetic acid was dissolved 1 g of N-[1-(R)-ethoxycarbonyl-3-phenylpropyl]-L-alanyl-N-(2-exonorbornyl)glycine tert-butyl ester, followed by addition of 2 ml of a 30% solution of hydrogen bromide in acetic acid. The mixture was allowed to stand at room temperature for 10 minutes. The reaction mixture was then shaken with 20 ml of ether and 50 ml of petroleum ether, and the organic layer was decanted off. The syrupy residue was crystallized from ether to give 1 g of N-[1-(R)-ethoxycarbonyl-3-phenylpropyl]-L-alanyl-N-(2-exonorbornyl)glycine hydrobromide as colorless prisms melting at 176—179°C.

Elemental analysis:
Calcd. for $C_{24}H_{34}N_2O_5 \cdot HBr$ : C, 56.36; H, 6.90; N, 5.48
Found : C, 56.35; H, 7.15; N, 5.22

$[\alpha]_D^{22}$ − 36.8° (c=1 in methanol)
Mass spectrum m/e: 412 (M—$H_2O$)

Example 17

In 4 ml of acetic acid was dissolved 2 g of N-[1-(S)-ethoxycarbonyl-3-phenylpropyl]-L-alanyl-N-(2-exonorbornyl)glycine tert-butyl ester, and in the same manner as Example 16, this solution was treated with 3 ml of a 30% solution of hydrogen bromide in acetic acid to give 1.8 g of N-[1-(2)-ethoxycarbonyl-3-phenylpropyl]-L-alanyl-N-(2-exonorbornyl)glycine hydrobromide as a colorless powdery product.

Elemental analysis:

Calcd. for $C_{24}H_{34}N_2O_5 \cdot HBr \cdot H_2O$ : C, 54.44; H, 7.04; N, 5.29
Found : C, 54.71; H, 7.12; N, 5.22

$[\alpha]_D^{25}$ −9.2° (c=1 in methanol)
Mass spectrum m/e: 412 (M—$H_2O$)

Example 18

In the same manner as Example 15, 1.1 g of L-alanyl-N-(2-adamantyl)glycine tert-butyl ester oxalate was reacted with 1.1 g of ethyl 4-phenyl-2-oxobutyrate to give 0.2 g of N-(1-ethoxycarbonyl-3-phenylpropyl)-L-alanyl-N-(2-adamantyl)glycine tert-butyl ester as an oil.

IR spectrum $\nu_{Neat}$ $cm^{-1}$: 1720, 1640 (C=O)
NMR spectrum ($CDCl_3$) $\delta$: 1.1—1.5 (m, 15H), 1.7—4.4 (m, 25H), 7.1 (s, 5H).

Example 19

In the same manner as Example 16, 0.2 g of N-(1-ethoxycarbonyl-3-phenylpropyl)-L-alanyl-N-(2-adamantyl)glycine tert-butyl ester was treated with a solution of hydrogen bromide in acetic acid to give 0.1 g of N-(1-ethoxycarbonyl-3-phenylpropyl)-L-alanyl-N-(2-adamantyl)glycine hydrobromide as a colorless powdery product.

Elemental analysis:

Calcd. for $C_{27}H_{38}N_2O_5 \cdot HBr \cdot 1/2H_2O$ : C, 57.85; H, 7.19; N, 5.00
Found : C, 57.59; H, 7.15; N, 4.64

Mass spectrum m/e: 452 (M—$H_2O$)

Example 20

In 8 ml of 50% ethanol was dissolved 0.2 g of L-alanyl-N-cyclopentylglycine and 0.83 g of 2-oxo-4-phenylbutanoic acid and, under stirring at room temperature, a solution of 0.17 g of sodium cyanoborohydride in 3 ml of methanol was added portionwise. The mixture was allowed to stand overnight and the solvent was distilled off, after which 10 ml of 10% aqueous sodium hydrogen carbonate was added. The oil separating out was removed by extraction with ethyl acetate, while the aqueous layer was neutralized and adsorbed on 20 ml of an ion exchange resin (IR—120B, $H^+$-form). After the column was washed with water, elution was carried out with 2% pyridine and the eluate was concentrated to give 0.2 g of N-(1-carboxy-3-phenylpropyl)-L-alanyl-N-cyclopentylglycine as crystals. This product was identical with the compound obtained in Example 13, and mixture-melting showed no depression of melting point.

Example 21

(1) Bicyclo[3.3.1]nonan-9-one (1.96 g) was allowed to react with glycine tert-butyl ester phosphite (3.66 g) in the presence of sodium cyanoborohydride in the same manner as in Reference Example 1 to give 2 g of N-[bicyclo[3.3.1]non-9-yl]glycine tert-butyl ester as a colorless oil.

IR spectrum $\nu_{max}^{neat}$ $cm^{-1}$: 2900, 1725, 1360, 1224, 1148.

(2) N-[Bicyclo[3.3.1]non-9-yl]glycine tert-butyl ester (2 g) was allowed to react with N-carbobenzoxy-L-alanine (4.6 g) in the same manner as in Reference Example 4 to give 4.8 g of N-carbobenzoxy-L-alanyl-N-[bicyclo[3.3.1]non-9-yl]-glycine tert-butyl ester as an oil.

IR spectrum $\nu_{max}^{neat}$ $cm^{-1}$: 1712, 1234, 1150, 1012.

(3) The above oil was subjected to catalytic reduction over palladium-charcoal in the same manner as in Reference Example 12 to give 2.1 g of L-alanyl-N-[bicyclo[3.3.1]non-9-yl]glycine tert-butyl ester oxalate as colorless solid foam.

(4) L-Alanyl-N-[bicyclo[3.3.1]non-9-yl]glycine tert-butyl ester oxalate (2.1 g) was allowed to react in the same manner as in Example 15. The obtained oily substance was purified by column chromatography on silica gel (ethyl acetate, n-hexane=1:2). The first eluate gave 0.13 g of N-[1-(R)-ethoxycarbonyl-3-phenylpropyl]-L-alanyl-N-[bicyclo[3.3.1]non-9-yl]glycine tert-butyl ester as an oily substance.

IR spectrum $\nu_{max}^{KBr}$ $cm^{-1}$: 2900, 1720, 1632, 1360, 1214, 1146.

(5) The second eluate gave 0.16 g of N-[1-(S)-ethoxycarbonyl-3-phenylpropyl]-L-alanyl-N-[bi-

cyclo[3.3.1]non-9-yl]glycine tert-butyl ester as an oil.

IR spectrum $\nu_{max}^{KBr}$ cm$^{-1}$: 2970, 1726, 1668, 1366, 1222, 1154.

Example 22

N-[1-(R)-Ethoxycarbonyl-3-phenylpropyl]-L-alanyl-N-[bicyclo[3.3.1]non-9-yl]glycine tert-butyl ester (0.13 g) was dissolved in 6N-hydrogen chloride-ethyl acetate (1.5 ml), and the mixture was allowed to stand at room temperature for 4 hours. After the solvent was evaporated and the residue was washed twice with ether, petroleum ether was poured onto the residue to afford a powdery substance, which was filtered immediately and dried to give 77 mg of N-[1-(R)-ethoxycarbonyl-3-phenylpropyl]-L-alanyl-N-[bicyclo[3.3.1]non-9-yl]glycine hydrochloride.

IR spectrum $\nu_{max}^{KBr}$ cm$^{-1}$: 2910, 1730, 1644, 1444, 1366, 1208.
Mass spectrum m/e: 440 ($M^+$—18), 336, 234 (base peak).

Example 23

Treatment of 0.16 g of N-[1-(S)-ethoxycarbonyl-3-phenylpropyl]-L-alanyl-N-[bicyclo[3.3.1]non-9-yl]glycine tert-butyl ester in the same manner as in Example 22 gave 0.137 g of N-[1-(S)-ethoxycarbonyl-3-phenylpropyl]-L-alanyl-N-[bicyclo[3.3.1]non-9-yl]glycine hydrochloride as a white powdery product.

IR spectrum $\nu_{max}^{KBr}$ cm$^{-1}$: 2910, 1730, 1668, 1534, 1206.
Mass spectrum m/e: 440 ($M^+$—18), 336, 319, 291, 234 (base peak).

Example 24

(1) trans-Bicyclo[4.3.0]nonan-8-one (4.1 g), glycine tert-butyl ester phosphite (8.2 g) and sodium cyanoborohydride (1.87 g) were allowed to react in the same manner as in Reference Example 1 to give 7.3 g of N-[trans-bicyclo[4.3.0]non-8-yl]glycine tert-butyl ester hydrochloride as colorless needles melting at 187—189°C.

(2) Four grams of N-[trans-bicyclo[4.3.0]non-8-yl]glycine tert-butyl ester hydrochloride was allowed to react with 4.4 g of N-carbobenzoxy-L-alanine in the same manner as in Reference Example 12 to give 4.5 g of L-alanyl-N-[trans-bicyclo[4.3.0]non-8-yl]glycine tert-butyl ester oxalate as a colorless powdery product.

$[\alpha]_D^{23}$ −3.3° (c=0.39, ethanol)

(3) The same reaction procedure as in Example 15 was conducted using 3 g of L-alanyl-N-[trans-bicyclo[4.3.0]non-8-yl]glycine tert-butyl ester oxalate, 3.2 g of ethyl 4-phenyl-2-oxobutyrate, 1.21 g of sodium acetate, 2.9 g of acetic acid, 5.8 g of molecular sieve 3A, 3.5 g of Raney nickel and 40 ml of ethanol, and the crude product was purified by column chromatography on silica gel (n-hexane, ethyl acetate=2:1). The first eluate gave 0.35 g of N-[1-(R)-ethoxycarbonyl-3-phenylpropyl]-L-alanyl-N-[trans-bicyclo[4.3.0]non-8-yl]glycine tert-butyl ester as an oil.

IR spectrum $\nu_{max}^{neat}$ cm$^{-1}$: 1740, 1640 (C=0).

(4) The second eluate gave 2.35 g of N-[1-(S)-ethoxycarbonyl-3-phenylpropyl]-L-alanyl-N-[trans-bicyclo[4.3.0]non-8-yl]glycine tert-butyl ester as an oil.

IR spectrum $\nu_{max}^{neat}$ cm$^{-1}$: 1740, 1640 (C=0).

Example 25

Treatment of 0.25 g of N-[1-(R)-ethoxycarbonyl-3-phenylpropyl]-L-alanyl-N-[trans-bicyclo-[4.3.0]non-8-yl)glycine tert-butyl ester in the same manner as in Example 22 gave 0.2 g of N-[1-(R)-ethoxycarbonyl-3-phenylpropyl]-L-alanyl-N-[trans-bicyclo[4.3.0]non-8-yl]glycine hydrochloride as colorless needles melting at 150—152°C.

Elemental analysis:

| | |
|---|---|
| Calcd. for $C_{26}H_{38}N_2O_5$·HCl | : C, 63.08; H, 7.94; N, 5.66 |
| Found | : C, 63.32; H, 7.99; N, 5.65 |

$[\alpha]_D^{24.5}$ −32.5° (c=1.01, ethanol).

Example 26

Treatment of 1.48 g of N-[1-(S)-ethoxycarbonyl-3-phenylpropyl]-L-alanyl-N-[trans-bicyclo-

[4.3.0]non-8-yl]glycine tert-butyl ester in the same manner as in Example 22 gave 1.29 g of N-[1-(S)-ethoxycarbonyl-3-phenylpropyl]-L-alanyl-N-[trans-bicyclo[4.3.0]non-8-yl]glycine hydrochloride as colorless crystals melting at 158—160°C.

Elemental analysis:
Calcd. for $C_{26}H_{38}N_2O_5{\cdot}HCl$ : C, 63.08; H, 7.94; N, 5.66
Found : C, 63.20; H, 8.12; N, 5.78

$[\alpha]_D^{24.5}$ 0° (c=0.95, ethanol).

Example 27

(1) cis-Bicyclo[4.3.0]nonan-8-one (5.95 g), glycine tert-butyl ester phosphite (11.8 g) and sodium cyanoborohydride (2.68 g) were allowed to react in the same manner as in Reference Example 1 to give 8.5 g of N-[cis-bicyclo[4.3.0]non-8-yl]glycine tert-butyl ester hydrochloride as colorless petals melting at 175—177°C.

(2) Four grams of N-[cis-bicyclo[4.3.0]non-8-yl]glycine tert-butyl ester hydrochloride was allowed to react with 4.4 g of N-carbobenzoxy-L-alanine in the same manner as Reference Example 12 to give 4.32 g of L-alanyl-N-[cis-bicyclo[4.3.0]non-8-yl]glycine tert-butyl ester oxalate as colorless crystals melting at 130—135°C.

$[\alpha]_D^{23}$ + 2.6° (c=1.0, ethanol)

(3) The same reaction procedure as in Example 15 was conducted using 4 g of L-alanyl-N-[cis-bicyclo[4.3.0]non-8-yl]glycine tert-butyl ester oxalate, 4.3 g of ethyl 4-phenyl-2-oxobutyrate, 1.6 g of sodium acetate, 3.9 g of acetic acid, 7.7 g of molecular sieve 3A, 4.7 g of Raney nickel and 55 ml of ethanol, and the crude product was purified by column chromatography on silica gel (n-hexane, ethyl acetate = 2:1). The first eluate gave 0.74 g of N-[1-(R)-ethoxycarbonyl-3-phenylpropyl]-L-alanyl-N-[cis-bicyclo[4.3.0]non-8-yl]glycine tert-butyl ester as an oil.

IR spectrum $\nu_{max}^{neat}$ $cm^{-1}$: 1735, 1645 (C=O).

(4) The second eluate gave 2.7 g of N-[1-(S)-ethoxycarbonyl-3-phenylpropyl]-L-alanyl-N-[cis-bicyclo[4.3.0]non-8-yl]glycine tert-butyl ester as an oil.

IR spectrum $\nu_{max}^{neat}$ $cm^{-1}$: 1740, 1640 (C=O).

Example 28

Treatment of 0.56 g of N-[1-(R)-ethoxycarbonyl-3-phenylpropyl]-L-alanyl-N-[cis-bicyclo[4.3.0]-non-8-yl]glycine tert-butyl ester in the same manner as in Example 22 gave 0.34 g of N-[1-(R)-ethoxycarbonyl-3-phenylpropyl]-L-alanyl-N-[cis-bicyclo[4.3.0]non-8-yl]glycine hydrochloride as colorless crystals melting at 114—115°C.

Elemental analysis:
Calcd. for $C_{26}H_{38}N_2O_5{\cdot}HCl$ : C, 63.08; H, 7.94; N, 5.66
Found : C, 62.95; H, 7.94; N, 5.66

$[\alpha]_D^{23}$ −31.7° (c=0.94, ethanol).

Example 29

Treatment of 1.5 g of N-[1-(S)-ethoxycarbonyl-3-phenylpropyl]-L-alanyl-N-[cis-bicyclo[4.3.0]-non-8-yl]glycine tert-butyl ester in the same manner as in Example 22 gave 1.2 g of N-[1-(S)-ethoxycarbonyl-3-phenylpropyl]-L-alanyl-N-[cis-bicyclo[4.3.0]non-8-yl]glycine hydrochloride as colorless needles melting at 142—144°C.

Elemental analysis:
Calcd. for $C_{26}H_{38}N_2O_5{\cdot}HCl{\cdot}2/5H_2O$: C, 62.17; H, 7.99; N, 5.58
Found : C, 62.29; H, 7.71; N, 5.53

$[\alpha]_D^{23}$ − 0.5° (c=1.02, ethanol).

Experiment 1
Inhibitions of Angiotensin I Converting Enzyme (ACE) by the Compounds of this Invention.

Experimental Method

The experiment was conducted in accordance with a modification of the method described by Cushman et al. [Biochemical Pharmacology, Vol. 20, 1637 (1971)]. That is, using hippuryl-L-histidyl-L-leucine (HHL) as the substrate, the ACE inhibitory activity was determined in terms of percent inhibition of the amount of hippuric acid produced by ACE when the present compound was added. A solution of the compound of the present invention dissolved in a 0.02 to 0.5% dimethyl sulfoxide-100 mM potassium phosphate buffer solution (pH 8.3, containing 300 mM sodium chloride) was added to 100 μl of ACE (protein concentration, 20 mg/ml) and 100 μl of 1.25 mM HHL. In this experiment, a potassium phosphate buffer solution containing dimethyl sulfoxide at a concentration equal to that of the test solution was used as a control. After incubating the solution at 37°C for one hour, 150 μl of 1N hydrochloric acid was added to the solution to terminate the reaction. After 1 ml of ethyl acetate was added, the solution was centrifuged at 3000 r.p.m. for 10 minutes. A 0.5 ml aliquot was separated from the ethyl acetate layer and dried at a temperature below 50°C under nitrogen gas streams. The residue was mixed with 5 ml of 1M aqueous sodium chloride and the mixture was subjected to colorimetry at a wavelength of 228 nm.

Test Result

The test results obtained with respect to the compounds of Examples 1, 2, 7, 13, 17 and 19 are shown in Table 1 below.

TABLE 1

| Example No. of Tested Compound | Concentration (μM) | Inhibitory Activity on ACE (%) |
|---|---|---|
| 1 | 1 | 84 |
| | 10 | 96 |
| 2 | 1 | 64 |
| | 10 | 90 |
| 7 | 1 | 88 |
| | 10 | 98 |
| 13 | 1 | 93 |
| | 10 | 100 |
| 17 | 1 | 78 |
| | 10 | 99 |
| 19 | 1 | 73 |
| | 10 | 93 |

Experiment 2

Effect of Present Compounds against Hypertensive Activity of Angiotensin I.

Experimental Method

Male rats (Sprague-Dawley) weighing 250 g to 350 g which were fed under free access to drinking water and feeds were used as experimental animals. The rats were anesthetized with intraperitoneal administration of pentobarbital sodium (50 mg/kg) on the day before the test day and a polyethylene tube was inserted into each of the femoral artery for measurement of blood pressure and the femoral vein for injection of angiotensin I and II, and then the tubes were fixed.

On the test day, an average blood pressure in the control phase was recorded on an electric hemodynamometer (MP—4T model manufactured by Nippon Koden, Japan) and thereafter angiotensin I and then angiotensin II were injected through the femoral vein at a dose of 300 ng/kg and 100 ng/kg respectively, to measure the hypertensive activity. Then, 13.8 $\mu$M/kg of the compound of this invention was administered orally as an aqueous solution or an aqueous gum arabic suspension, and 20, 60 and 120 minutes after the administration, angiotensin I and II were injected repeatedly to trace hypertensive reactions. In calculating the percent inhibition to the hypertensive activity of angiotensin I, the percent inhibitory value was corrected based on the variation with time in the hypertensive reaction by angiotensin II.

Test Result

The test results obtained with respect to the compounds of Examples 1, 2, 12 and 13 are shown in Table 2 below.

TABLE 2

| Example No. of Tested Compound | Percent Inhibition (%) against Hypertensive Reaction by Angiotensin I | | |
|---|---|---|---|
| | After 20 min. | After 60 min. | After 120 min. |
| 1 | 92 | 82 | 82 |
| 2 | 82 | 82 | 77 |
| 12 | 95 | 85 | 76 |
| 13 | 58 | 64 | 62 |

Preparation Example

The compounds (I) of the present invention are used, for example, for the treatment of hypertension in the following examples of formulation

1. Tablets

| | | |
|---|---|---|
| (1) | N-(1-Ethoxycarbonyl-3-phenylpropyl)-L-alanyl-N-cyclopentylglycine hydrochloride | 10 g |
| (2) | Lactose | 90 g |
| (3) | Corn Starch | 29 g |
| (4) | Magnesium Stearate | 1 g |
| | for 1000 tablets | 130 g |

The above ingredients (1), (2) and 17 g of corn starch are blended, and granulated using a paste prepared from 7 g of corn starch. Five grams of corn starch and the ingredient (4) are added to the resulting granules and the mixture is compressed by a tabletting machine to prepare 1000 tablets having a diameter 7 mm each containing 10 mg of the active ingredient (1).

2. Capsules

| | | |
|---|---|---|
| (1) | N-[1-(S)-Ethoxycarbonyl-3-phenylpropyl]-L-alanyl-N-(2-exonorbornyl)glycine hydrochloride | 10 g |
| (2) | Lactose | 135 g |
| (3) | Celulose Fine Powder | 70 g |
| (4) | Magnesium Stearate | 5 g |
| | for 1000 capsules | 220 g |

All of the above components are blended and encapsulated into Gelatin Capsule No. 3 (IX) Japanese Pharmacopoiea) to prepare 1000 capsules each containing 10 mg of the active component (1).

3. Injectable Solution

| | | |
|---|---|---|
| (1) | N-(1-Carboxy-3-phenylpropyl)-L-alanyl-N-cyclopentylglycine | 10 g |
| (2) | Sodium Chloride | 9 g |
| (3) | Chlorobutanol | 5 g |
| (4) | Sodium Bicarbonate | 1 g |

All of the above ingredients are dissolved in 1000 ml of distilled water and charged into 1000 brown ampules each containing 1 ml of the solution. The ampules are replaced with nitrogen gas and sealed. The entire preparation steps are conducted under sterile conditions.

**Claims**

1. An alicyclic compound of the formula

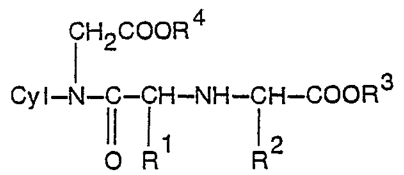

(I)

wherein Cyl is an alicyclic group containing up to 10 carbon atoms,
$R^1$ is hydrogen, lower alkyl or aralkyl,
$R^2$ is hydrogen, lower alkyl or aralkyl which may optionally be substituted, and
$R^3$ and $R^4$ are the same or different and each is hydrogen or lower alkyl,
or a pharmaceutically acceptable salt thereof.

2. A compound according to Claim 1, wherein Cyl represents $C_{3-10}$ alicyclic group, $R^1$ is hydrogen, $C_{1-4}$ alkyl or phenyl-$C_{1-2}$ alkyl, $R^2$ is hydrogen, $C_{1-4}$ alkyl or phenyl-$C_{1-4}$ alkyl which is unsubstituted or substituted by one to three of halogen, $C_{1-4}$ alkyl, $C_{1-4}$ alkoxy and hydroxyl, and $R^3$ and $R^4$ independently represent hydrogen or $C_{1-4}$ alkyl.

3. A compound according to Claim 2, wherein Cyl is $C_{4-7}$ alicyclic group, $R^1$ is hydrogen or $C_{1-4}$ alkyl, $R^2$ is hydrogen, $C_{1-4}$ alkyl or $C_{7-10}$ aralkyl, $R^3$ is hydrogen or $C_{1-4}$ alkyl, and $R^4$ is hydrogen.

4. A compound according to Claim 2, wherein the $C_{3-10}$ alicyclic group Cyl is $C_{3-8}$ cycloalkyl, or bi- or tricyclic $C_{7-10}$ alicyclic group.

5. A compound according to Claim 4, wherein the bi or tricyclic $C_{7-10}$ alicyclic group is norbornyl, bicyclooctyl, bicyclononyl or adamantyl.

6. A compound according to Claim 2, wherein $R^1$ is methyl or benzyl.

7. A compound according to Claim 2, wherein $R^2$ is phenethyl which is unsubstituted or substituted by one to three of $C_{1-4}$ alkyl.

8. A compound according to Claim 2, wherein $R^4$ is hydrogen.

9. The compound according to Claim 1, which is N-(1-ethoxycarbonyl-3-phenylpropyl)-L-alanyl-N-cyclopentylglycine.

10. The compound according to Claim 1, which is N-(1-butoxycarbonyl-3-phenylpropyl)-L-alanyl-N-cyclopentylglycine.

11. The compound according to Claim 1, which is N-(1-carboxy-3-phenylpropyl)-L-alanyl-N-cyclopentylglycine.

12. The compound according to Claim 1, which is N-(1-ethoxycarbonyl-3-phenylpropyl)-L-alanyl-N-(2-exonorbornyl)glycine.

13. The compound according to Claim 1, which is N-(1-ethoxycarbonyl-3-phenylpropyl)-L-alanyl-N-[bicyclo[4.3.0]non-8-yl]glycine.

14. The compound according to Claim 1, which is N-(1-ethoxycarbonyl-3-phenylpropyl)-L-alanyl-N-cycloheptylglycine.

15. A pharmaceutical composition which comprises, as an active ingredient, an effective amount of a compound as claimed in any one of Claims 1 to 14 in association with a pharmaceutically acceptable carrier, excipient or diluent therefor.

16. A compound as claimed in any one of Claims 1 to 14 or a pharmaceutical composition as claimed in Claim 15 for use in therapeutical treatment of a warm-blooded animal.

17. A process for producing an alicyclic compound (I) as claimed in Claim 1, which comprises

a) subjecting a compound of the formula (II):

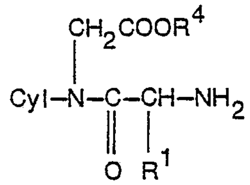

(II)

wherein all the symbols are as defined above, and a compound of the formula (III):

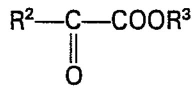

(III)

wherein $R^2$ and $R^3$ are as defined above, to condensation under reductive conditions, or

b) hydrolyzing an ester compound of the formula (I) wherein $R^3$ and/or $R^4$ is lower alkyl and all other symbols are as defined above, to provide a compound of the formula (I) wherein $R^3$ and/or $R^4$ is hydrogen and all other symbols are as defined above, or

c) subjecting a benzyl ester of a compound of the formula (I) wherein $R^3$ and/or $R^4$ is benzyl and all other symbols are as defined above, to catalytic reduction to provide a compound of the formula (I) wherein $R^3$ and/or $R^4$ is hydrogen and all other symbols are as defined above, or

d) subjecting a compound of the formula (IV):

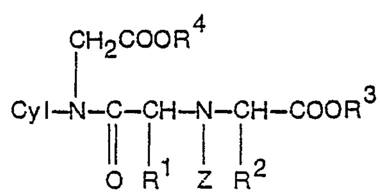

(IV)

wherein Z is a protective group removable by hydrolysis or catalytic reduction and the other symbols are as defined above, to hydrolysis or catalytic reduction, or

e) reacting a compound of the formula (II) with a compound of the formula (V):

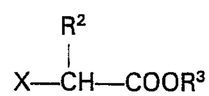

(V)

wherein $R^2$ and $R^3$ are as defined above, and X is halogen or a group of the formula $R^5SO_2$—O— in which $R^5$ is $C_{1-4}$ alkyl, phenyl or p-tolyl, and

f) if desired, converting the thus obtained compound of the formula (I) into a pharmaceutically acceptable salt thereof.

**Patentansprüche**

1. Alicyclische Verbindung der Formel

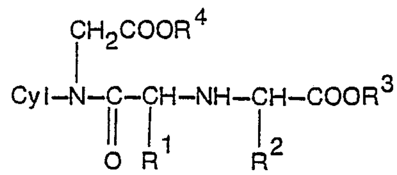

(I)

in der

Cyl eine alicyclische Gruppe mit bis zu 10 Kohlenstoff-Atomen ist,
$R^1$ Wasserstoff, Niederalkyl oder Aralkyl ist,
$R^2$ Wasserstoff, Niederalkyl oder Aralkyl, das gegebenenfalls substituiert sein kann, ist,
$R^3$ und $R^4$ gleich oder verschieden und jeweils Wasserstoff oder Niederalkyl sind,
oder ein pharmazeutisch unbedenkliches Salz derselben.

2. Verbindung nach Anspruch 1, dadurch gekennzeichnet, daß
Cyl für eine alicyclische $C_{3-10}$-Gruppe steht,
$R^1$ Wasserstoff, $C_{1-4}$-Alkyl oder Phenyl-$C_{1-2}$-alkyl ist,
$R^2$ Wasserstoff, $C_{1-4}$-Alkyl oder Phenyl-$C_{1-4}$-alkyl ist, das unsubstituiert oder durch ein bis drei Substituenten Halogen, $C_{1-4}$-Alkyl, $C_{1-4}$-Alkoxy und Hydroxyl substituiert ist, und
$R^3$ und $R^4$ unabhängig voneinander für Wasserstoff oder $C_{1-4}$-Alkyl stehen.

3. Verbindung nach Anspruch 2, dadurch gekennzeichnet, daß
Cyl eine alicyclische $C_{4-7}$-Gruppe ist,
$R^1$ Wasserstoff oder $C_{1-4}$-Alkyl ist,
$R^2$ Wasserstoff, $C_{1-4}$-Alkyl oder $C_{7-10}$-Aralkyl ist,
$R^3$ Wasserstoff oder $C_{1-4}$-Alkyl ist und
$R^4$ Wasserstoff ist.

4. Verbindung nach Anspruch 2, dadurch gekennzeichnet, daß die alicyclische $C_{3-10}$-Gruppe Cyl $C_{3-8}$-Cycloalkyl oder eine bi- oder tricyclische alicyclische $C_{7-10}$-Gruppe ist.

5. Verbindung nach Anspruch 4, dadurch gekennzeichnet, daß die bi- oder tricyclische alicyclische $C_{7-10}$-Gruppe Norbornyl, Bicyclooctyl, Bicyclononyl oder Adamantyl ist.

6. Verbindung nach Anspruch 2, dadurch gekennzeichnet, daß $R^1$ Methyl oder Benzyl ist.

7. Verbindung nach Anspruch 2, dadurch gekennzeichnet, daß $R^2$ Phenethyl ist, das unsubstituiert oder durch ein bis drei $C_{1-4}$-Alkyl substituiert ist.

8. Verbindung nach Anspruch 2, dadurch gekennzeichnet, daß $R^4$ Wasserstoff ist.

9. Verbindung N-(1-Ethoxycarbonyl-3-phenylpropyl)-L-alanyl-N-cyclopentylglycin nach Anspruch 1.

10. Verbindung N-(1-Butoxycarbonyl-3-phenylpropyl)-L-alanyl-N-cyclopentylglycin nach Anspruch 1.

11. Verbindung N-(1-Carboxy-3-phenylpropyl)-L-alanyl-N-cyclopentylglycin nach Anspruch 1.

12. Verbindung N-(1-Ethoxycarbonyl-3-phenylpropyl)-L-alanyl-N-(2-exo-norbornyl)glycin nach Anspruch 1.

13. Verbindung N-(1-Ethoxycarbonyl-3-phenylpropyl)-L-alanyl-N-(bicyclo[4.3.0]non-8-yl)glycin nach Anspruch 1.

14. Verbindung N-(1-Ethoxycarbonyl-3-phenylpropyl)-L-alanyl-N-cycloheptylglycin nach Anspruch 1.

15. Pharmazeutische Zusammensetzung, enthaltend als Wirkstoff eine wirksame Menge einer Verbindung nach irgendeinem der Ansprüche 1 bis 14 in Verbindung mit einem pharmazeutisch unbedenklichen Träger, Exzipienten oder Verdünnungsmittel für diese.

16. Verbindung nach irgendeinem der Ansprüche 1 bis 14 oder einer pharmazeutischen Zusammensetzung nach Anspruch 15 zur Verwendung bei der therapeutischen Behandlung eines warmblütigen Tieres.

17. Verfahren zur Herstellung einer alicyclischen Verbindung der Formel (I) nach Anspruch 1, dadurch gekennzeichnet, daß

a) eine Verbindung der Formel (II)

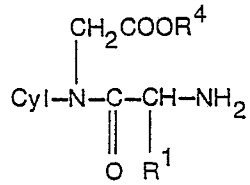

(II)

in der sämtliche Symbole die im Vorstehenden angegebenen Bedeutungen haben, und eine Verbindung der Formel (III)

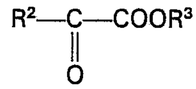 (III),

in der $R^2$ und $R^3$ die im Vorstehenden angegebenen Bedeutungen haben, der Kondensation unter reduzierenden Bedingungen unterworfen werden, oder

b) eine Ester-Verbindung der Formel (I), in der $R^3$ und/oder $R^4$ Niederalkyl sind und sämtliche anderen Symbole die im Vorstehenden angegebenen Bedeutungen haben, hydrolysiert wird, wodurch eine Verbindung der Formel (I) erhalten wird, in der $R^3$ und/oder $R^4$ Wasserstoff sind und sämtliche anderen Symbole die im Vorstehenden angegebenen Bedeutungen haben, oder

c) ein Benzyl-Ester einer Verbindung der Formel (I), in der $R^3$ und/oder $R^4$ Benzyl sind und sämtliche anderen Symbole die im Vorstehenden angegebenen Bedeutungen haben, der katalytischen Reduktion unterworfen wird, wodurch eine Verbindung der Formel (I) erhalten wird, in der $R^3$ und/oder $R^4$ Wasserstoff sind und sämtliche anderen Symbole die im Vorstehenden angegebenen Bedeutungen haben, oder

d) eine Verbindung der Formel

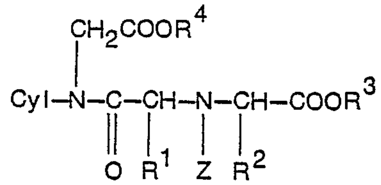 (IV)

in der Z eine durch Hydrolyse oder durch katalytische Reduktion abspaltbare Schutzgruppe ist und die anderen Symbole die im Vorstehenden angegebenen Bedeutungen haben, der Hydrolyse oder der katalytischen Reduktion unterworfen wird, oder

e) eine Verbindung der Formel (II) mit einer Verbindung der Formel (V)

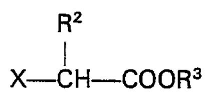 (V),

in der $R^2$ und $R^3$ die im Vorstehenden angegebenen Bedeutungen haben und X Halogen oder eine Gruppe der Formel $R^5SO_2$—O— ist, worin $R^5$ $C_{1-4}$-Alkyl, Phenyl oder p-Tolyl ist, umgesetzt wird und

f) die so erhaltene Verbindung der Formel (I) gewünschtenfalls in ein pharmazeutisch unbedenkliches Salz derselben überführt wird.

**Revendications**

1. Composé alicylique ayant la formule:

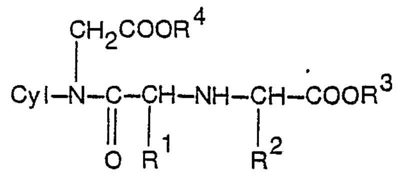 (I)

dans laquelle Cyl est un groupe alicyclique contenant jusqu'à 10 atomes de carbone

$R^1$ est de l'hydrogène, un groupe alkyle inférieur ou arylalkyle,

$R^2$ de l'hydrogène, un groupe alkyle inférieur ou arylalkyle qui peut être éventuellement substitué, et

$R^3$ et $R^4$ sont identiques ou différents et chacun est de l'hydrogène ou un groupe alkyle inférieur, ou un sel de ce composé pharmaceutiquement acceptable.

2. Composé selon la revendication 1, dans lequel Cyl représente un groupe $C_{3-10}$ alicyclique; $R^1$ est de l'hydrogène, un groupe $C_{1-4}$ alkyle ou phényl-$C_{1-2}$ alkyle; $R^2$ est de l'hydrogène, un groupe $C_{1-4}$ alkyle ou phényl-$C_{1-4}$ alkyle qui est non substitué ou substitué par un à trois des radicaux suivants halogéno-$C_{1-4}$ alkyle, $C_{1-4}$ alcoxy et hydroxyle, et $R^3$ et $R^4$ représentent indépendamment de l'hydrogène ou un groupe $C_{1-4}$ alkyle.

3. Composé selon la revendication 2, dans lequel Cyl est un groupe $C_{4-7}$ alicyclique; $R^1$ est de

l'hydrogène ou un groupe $C_{1-4}$ alkyle; $R^2$ est de l'hydrogène un groupe $C_{1-4}$ alkyle ou $C_{7-10}$ arylalkyle; $R^3$ est de l'hydrogène ou un groupe $C_{1-4}$ alkyle, et $R^4$ est de l'hydrogène.

4. Composé selon la revendication 2, dans lequel le groupe $C_{3-10}$ alicyclique Cyl est un groupe $C_{3-8}$ cycloalkyle ou un groupe $C_{7-10}$ alicyclique bi- ou tricyclique.

5. Composé selon la revendication 4, dans lequel le groupe $C_{7-10}$ alicyclique bi ou tricyclique est un groupe norbornyl bicyclooctyle, bicyclononyle ou adamantyle.

6. Composé selon la revendication 2, dans lequel $R^1$ est un groupe méthyle ou benzyle.

7. Composé selon la revendication 2, dans lequel $R^2$ est un groupe phénéthyle qui est non substitué ou substitué par un à trois radicaux $C_{1-4}$ alkyle.

8. Composé selon la revendication 2, dans lequel $R^4$ est de l'hydrogène.

9. Composé selon la revendication 1, qui est la N-(1-éthoxycarbonyl-3-phénylpropyl)-L-alanyl-N-cyclopentylglycine.

10. Composé selon la revendication 1, qui est la N-(1-butoxycarbonyl-3-phénylpropyl)-L-alanyl-N-cyclopentylglycine.

11. Composé selon la revendication 1, qui est la N-(1-carboxy-3-phénylpropyl)-L-alanyl-N-cyclopentylglycine.

12. Composé selon la revendication 1, qui est la N-(1-éthoxycarbonyl-3-phénylpropyl)-L-alanyl-N-(2-exonorbornyl)-glycine.

13. Composé selon la revendication 1, qui est la N-(1-éthoxycarbonyl-3-phénylpropyl)-L-alanyl-N-[bicyclo[4,3,0]non-8-yl]glycine.

14. Composé selon la revendication 1, qui est la N-(1-éthoxycarbonyl-3-phénylpropyl)-L-alanyl-N-cycloheptylglycine.

15. Composition pharmaceutique qui comprend, comme ingrédient actif, une quantité efficace d'un composé tel que revendiqué dans l'une quelconque des revendications 1 à 14 en association avec un véhicule, excipient ou diluant pour ce composé pharmaceutiquement acceptable.

16. Composé selon l'une quelconque des revendications 1 à 14 ou composition pharmaceutique selon la revendication 15 pour usage en traitement thérapeutique d'un animal à sang chaud.

17. Procédé pour la préparation d'un composé alicyclique (I) selon la revendication 1 qui consiste

a) à soumettre un composé de formule (II):

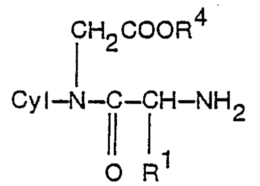

(II)

dans laquelle tous les symboles ont été définis ci-dessus, et un composé de formule (III):

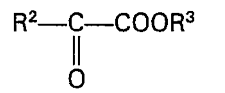

(III)

dans laquelle $R^2$ et $R^3$ sont définis ci-dessus, à une condensation dans des conditions réductrices, ou

b) à hydrolyser un composé ester de formule (I) dans lequel $R^3$ et/ou $R^4$ est un groupe alkyle inférieur et tous les autres symboles sont définis ci-dessus, pour donner un composé de formule (I) dans laquelle $R^3$ et/ou $R^4$ est de l'hydrogène et tous les autres symboles sont comme définis ci-dessus, ou

c) à soumettre un ester benzylique d'un composé de formule (I) dans lequel $R^3$ et/ou $R^4$ est un groupe benzyle et tous les autres symboles sont tels que définis ci-dessus, a une réduction catalytique pour donner un composé de formule (I) dans lequel $R^3$ et/ou $R^4$ est de l'hydrogène et tous les autres symboles sont tels que définis ci-dessus

d) à soumettre un composé de formule (IV):

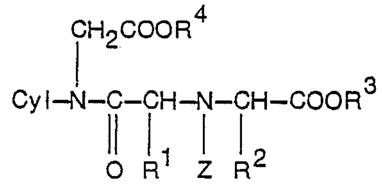

(IV)

dans lequel Z est un groupe protectuer éliminable par l'hydrolyses ou par réduction catalytique et les autres symboles sont tels que définis ci-dessus, à l'hydrolyse ou à une réduction catalytique, ou bien

e) à faire réagir un composé de formule (II) avec un composé de formule (V):

$$\begin{array}{l} \quad\;\; R^2 \\ \quad\;\; | \\ X—CH—COOR^3 \end{array} \qquad (V)$$

dans laquelle $R^2$ et $R^3$ sont tels que définis ci-dessus, et X est un atome d'halogène ou un groupe de formule $R^5SO_2$—O— dans laquelle $R^5$ est un group $C_{1-4}$ alkyle, phényle ou p-tolyle, et

f) si on le désire, à convertir le composé ainsi obtenu de formule (I) en un de ses sels pharmaceutiquement acceptables.